(19) Europäisches Patentamt
European Patent Office
Office européen des brevets

(11) EP 4 477 699 A1

(12) **EUROPEAN PATENT APPLICATION**
published in accordance with Art. 153(4) EPC

(43) Date of publication:
**18.12.2024 Bulletin 2024/51**

(21) Application number: **23752843.5**

(22) Date of filing: **06.02.2023**

(51) International Patent Classification (IPC):
***C08J 11/18*** (2006.01) ***B01J 31/22*** (2006.01) ***B01J 31/24*** (2006.01) ***C08C 19/08*** (2006.01) ***C08J 11/12*** (2006.01)

(52) Cooperative Patent Classification (CPC):
**B01J 31/22; B01J 31/24; C08C 19/08; C08J 11/12; C08J 11/18;** Y02W 30/62

(86) International application number:
**PCT/JP2023/003873**

(87) International publication number:
**WO 2023/153379 (17.08.2023 Gazette 2023/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.02.2022 JP 2022018304**

(71) Applicants:
- **BRIDGESTONE CORPORATION**
**Chuo-ku**
**Tokyo 104-8340 (JP)**
- **Tohoku University**
**Sendai-shi, Miyagi 980-8577 (JP)**
- **National Institute of Advanced Industrial Science and Technology**
**Chiyoda-ku**
**Tokyo 100-8921 (JP)**

(72) Inventors:
- **HOJO Masahiro**
**Tokyo 104-8340 (JP)**
- **OKUNO Akira**
**Tokyo 104-8340 (JP)**
- **KUNO Marino**
**Tokyo 104-8340 (JP)**
- **YOSHIOKA Toshiaki**
**Sendai-shi, Miyagi 980-8577 (JP)**
- **KUMAGAI Shogo**
**Sendai-shi, Miyagi 980-8577 (JP)**
- **FUKAYA Norihisa**
**Tsukuba-shi, Ibaraki 305-8561 (JP)**
- **WAHYU Satpriyo Putro**
**Tsukuba-shi, Ibaraki 305-8561 (JP)**
- **CHOI Jun-Chul**
**Tsukuba-shi, Ibaraki 305-8561 (JP)**
- **MIFTAH Faried**
**Tsukuba-shi, Ibaraki 305-8561 (JP)**
- **YAMASHITA Hiroshi**
**Tsukuba-shi, Ibaraki 305-8561 (JP)**
- **FUJITANI Tadahiro**
**Tsukuba-shi, Ibaraki 305-8561 (JP)**
- **HATORI Makiko**
**Tsukuba-shi, Ibaraki 305-8561 (JP)**
- **SUGIKI Makoto**
**Tsukuba-shi, Ibaraki 305-8561 (JP)**

(74) Representative: **Marks & Clerk LLP**
**15 Fetter Lane**
**London EC4A 1BW (GB)**

# (54) METHOD FOR DECOMPOSING CROSSLINKED RUBBER

(57) The present disclosure addresses the problem of providing a method for decomposing a crosslinked rubber that can improve the yield of monomers. The solution is a method of decomposing a crosslinked rubber that includes: a first decomposition step of decomposing a crosslinked rubber containing a diene rubber, using a catalyst represented by the following general formula (1), (2), or (3), where M is ruthenium, molybdenum, or the like, $X^1$, $X^2$, $L^1$, $L^2$, and $L^3$ each independently represent a ligand, $R^1$, $R^2$, and $R^3$ each independently represent hydrogen, an alkyl group, a cycloalkyl group, an alkenyl group, an alkynyl group, an aryl group, or the like (these groups may be substituted by one or more alkyl groups, halogens, alkoxy groups, or the like), $L^1$ and $L^2$, $R^1$ and $R^2$, and $L^1$ and $R^1$ may respectively bond with each other to form rings; and a second decomposition step of pyrolyzing a decomposition product obtained by the first decomposition step under an inert gas atmo-

EP 4 477 699 A1

sphere and in the absence of a catalyst at a temperature of 300°C to 450°C.

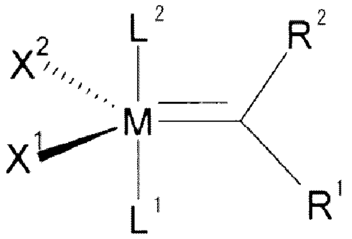
... (1)

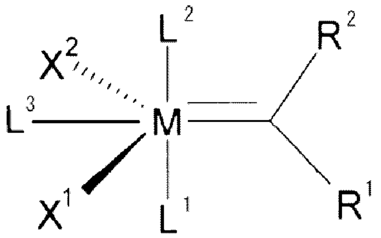
... (2)

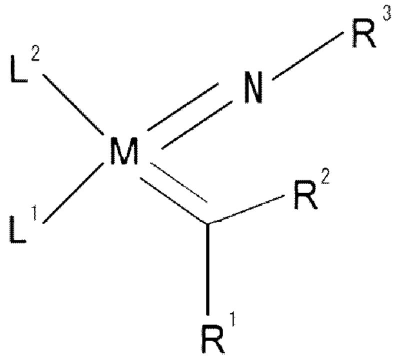
... (3)

## Description

### TECHNICAL FIELD

The present disclosure relates to a method of decomposing a crosslinked rubber.

### BACKGROUND

Conventionally, rubber products made primarily from crosslinked rubber such as vulcanized rubber are difficult to reuse and are often reused as fuel after the life of a product, particularly in cement plants and the like. However, in recent years, with the growing concern about environmental issues, there is a demand for development of methods to reuse materials obtained by decomposing rubber products instead of burning rubber products as fuel.

There are various methods for decomposing a crosslinked rubber. For example, a technique of pyrolysis of crosslinked rubber at high temperatures is known. Further, Patent Literature (PTL) 1 describes a method of decomposing polyisoprene rubber by microorganisms.

### CITATION LIST

#### Patent Literature

PTL 1: JP 2009-247241 A

### SUMMARY

#### (Technical Problem)

As mentioned above, there are various methods for decomposing a crosslinked rubber. From the viewpoint of further improving the recyclability of crosslinked rubber, it is important to increase the yield of monomers obtained through decomposition.

However, when crosslinked rubber is pyrolyzed at high temperature as described above, decomposition products gasify or aromatize, resulting in lower yields of monomers. Further, when using microorganisms to decompose crosslinked rubber, as in the technology described in PTL 1, a long time is required for decomposition, and further, the yield of monomers is low.

It would be helpful to solve the problems of the conventional technologies described above and to provide a method of decomposing a crosslinked rubber that can improve the yield of monomers.

#### (Solution to Problem)

Primary features of a method of decomposing a crosslinked rubber according to the present disclosure, as a solution to the problems described above, are as follows.

[1] A method of decomposing a crosslinked rubber, the method comprising:

a first decomposition step of decomposing a crosslinked rubber containing a diene rubber, using a catalyst represented by the following general formula (1), (2), or (3):

[Chem. 1]

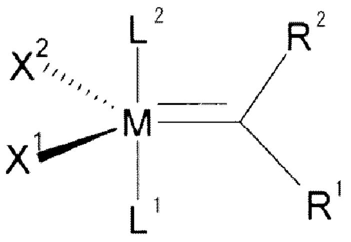

··· (1)

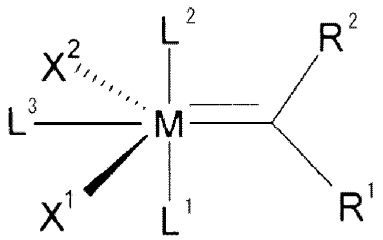

··· (2)

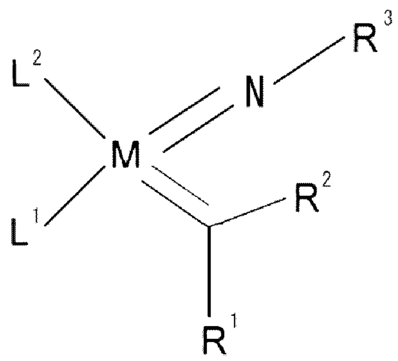

··· (3)

wherein, in the formulas, M is ruthenium, titanium, molybdenum, or tungsten,
$X^1$ and $X^2$ each independently represent a ligand,
$L^1$, $L^2$, and $L^3$ each independently represent a ligand,
$R^1$, $R^2$, and $R^3$ each independently represent hydrogen, an alkyl group, a cycloalkyl group, an alkenyl group, an alkynyl group, an aryl group, a carboxylate group, an alkoxy group, an alkenyloxy group, an alkynyloxy group, an aryloxy group, an alkoxycarbonyl group, an alkylamino group, an alkylthio group, an arylthio group, an alkylsulfonyl group, or an alkylsulfinyl group, wherein these groups may be substituted by one or more alkyl groups, halogens, alkoxy groups, aryl groups, or heteroaryl groups,
$L^1$ and $L^2$ may bond with each other to form a ring,
$R^1$ and $R^2$ may bond with each other to form a ring, and
$L^1$ and $R^1$ may bond with each other to form a ring; and
a second decomposition step of pyrolyzing a decomposition product obtained by the first decomposition step under an inert gas atmosphere and in the absence of a catalyst at a temperature of 300°C or more and 450°C or less.

[2] The method of decomposing a crosslinked rubber according to [1], wherein a rubber component of the crosslinked rubber contains an isoprene unit and/or a butadiene unit,

the total fraction of the isoprene unit and the butadiene unit in the rubber component is 40 mass% or more, total mass, A, of the isoprene unit and the butadiene unit in the rubber component and mass, B, of aromatic compounds derived from the isoprene unit and/or the butadiene unit in the rubber component of the decomposition product obtained by the first decomposition step satisfy the relationship of the following expression (1):

$$B/A \times 100 \leq 50\ (\text{mass\%}) \cdots (1)$$

wherein A is the total mass of the isoprene unit and the butadiene unit in the rubber component of the crosslinked rubber, and B is the mass of the aromatic compounds derived from the isoprene unit and/or the butadiene unit in the rubber component of the crosslinked rubber, in the decomposition product obtained by the first decomposition step.

[3] The method of decomposing a crosslinked rubber according to [1] or [2], wherein a liquid component of the decomposition product obtained in the first decomposition step is oligomers having a weight-average molecular weight of 100 to 50,000.
[4] The method of decomposing a crosslinked rubber according to any one of [1] to [3], wherein a decomposition product obtained by the second decomposition step contains 15 mass% or more of a hydrocarbon compound having 5 or less carbon atoms and limonene.
[5] The method of decomposing a crosslinked rubber according to any one of [1] to [4], wherein the first decomposition step is performed at a temperature of 20°C or more and 200°C or less.
[6] The method of decomposing a crosslinked rubber according to any one of [1] to [5], wherein the diene rubber includes at least one rubber selected from the group consisting of isoprene skeleton rubber, styrene-butadiene rubber, and butadiene rubber.
[7] The method of decomposing a crosslinked rubber according to any one of [1] to [6], wherein the crosslinked rubber further contains carbon black.
[8] The method of decomposing a crosslinked rubber according to any one of [1] to [7], wherein the crosslinked rubber further contains sulfur.

(Advantageous Effect)

The present disclosure provides a method of decomposing a crosslinked rubber that can improve the yield of monomers.

DETAILED DESCRIPTION

The following is a detailed illustrative description of a method of decomposing a crosslinked rubber based on an embodiment of the present disclosure.
The method of decomposing a crosslinked rubber includes:

a first decomposition step of decomposing a crosslinked rubber containing a diene rubber, using a catalyst represented by the following general formula (1), (2), or (3):

[Chem. 2]

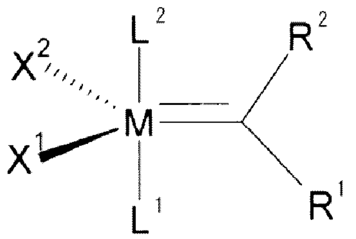

... (1)

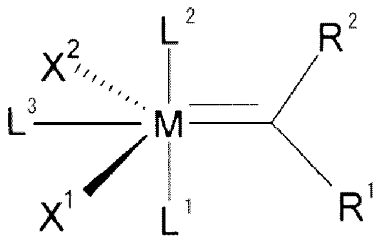

... (2)

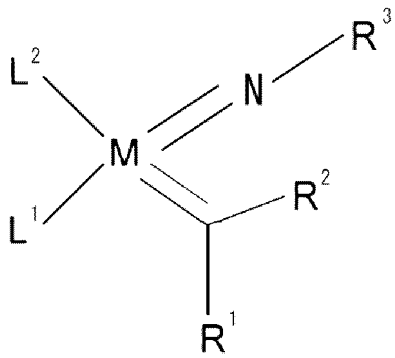

... (3)

wherein, in the formulas, M is ruthenium, titanium, molybdenum, or tungsten,

$X^1$ and $X^2$ each independently represent a ligand,

$L^1$, $L^2$, and $L^3$ each independently represent a ligand,

$R^1$, $R^2$, and $R^3$ each independently represent hydrogen, an alkyl group, a cycloalkyl group, an alkenyl group, an alkynyl group, an aryl group, a carboxylate group, an alkoxy group, an alkenyloxy group, an alkynyloxy group, an aryloxy group, an alkoxycarbonyl group, an alkylamino group, an alkylthio group, an arylthio group, an alkylsulfonyl group, or an alkylsulfinyl group, wherein these groups may be substituted by one or more alkyl groups, halogens, alkoxy groups, aryl groups, or heteroaryl groups,

$L^1$ and $L^2$ may bond with each other to form a ring,

$R^1$ and $R^2$ may bond with each other to form a ring, and

$L^1$ and $R^1$ may bond with each other to form a ring; and

a second decomposition step of pyrolyzing a decomposition product obtained by the first decomposition step under an inert gas atmosphere and in the absence of a catalyst at a temperature of 300°C or more and 450°C or less.

The catalyst represented by the above general formula (1), (2), or (3) used in the first decomposition step of the method of decomposing a crosslinked rubber can promote metathesis decomposition, and metathesis decomposition occurs at unsaturated bonds in the diene rubber to decompose the diene rubber. Here, unlike ordinary pyrolysis, metathesis decomposition does not require high temperatures, and therefore diene rubber can be decomposed under

mild conditions. Further, metathesis decomposition can suppress aromatization of decomposition products and improve the retention rate of monomer skeletons (isoprene skeleton, butadiene skeleton, and the like) in diene rubber compared to ordinary pyrolysis.

Further, in the method of decomposing a crosslinked rubber, in the second decomposition step, decomposition product obtained in the first decomposition step is pyrolyzed under an inert gas atmosphere and in the absence of a catalyst at a temperature of 300°C or more and 450°C or less, thereby inhibiting cyclization and aromatization of double bonds in monomer skeletons and oxidation, so that the decomposition product (intermediate decomposition product) can be decomposed into monomers (in particular, diene monomers).

Accordingly, the method of decomposing a crosslinked rubber may improve the yield of monomers ultimately obtained from the crosslinked rubber.

<First decomposition step>

The method of decomposing a crosslinked rubber according to the present embodiment includes a first decomposition step of decomposing a crosslinked rubber containing a diene rubber using a catalyst represented by the above general formula (1), (2), or (3).

(Crosslinked rubber)

The crosslinked rubber that is the decomposition target of the method according to the present embodiment contains diene rubber and may further contain carbon black, sulfur, and the like.

The form of the crosslinked rubber is not particularly limited and may be, for example, powdered rubber. Rubber powder can be obtained by shredding and granulating used rubber products such as waste tires. A granulating step may include multiple steps, such as a preliminary granulating step and a fine granulating step, or particle size of powdered rubber used may be adjusted after the granulating step via a sorting step.

-- Diene rubber --

The diene rubber is a rubber containing units derived from diene monomers (diene units) and may further contain units derived from copolymerizable comonomers.

The diene monomer-derived units described above enable crosslinking (vulcanization) of diene rubber and also enable diene rubber to develop rubber-like elongation and strength. In crosslinked rubber, diene rubber is normally present in a crosslinked state, but some diene rubber may be not crosslinked. Diene monomers (diene compounds) specifically include 1,3-butadiene, isoprene, 1,3-pentadiene, 2,3-dimethyl-1,3-butadiene, and the like.

On the other hand, aromatic vinyl compounds and the like are examples of copolymerizable comonomers. Examples of aromatic vinyl compounds include styrene, o-methylstyrene, m-methylstyrene, p-methylstyrene, o,p-dimethylstyrene, o-ethylstyrene, m-ethylstyrene, p-ethylstyrene, and the like.

Further, examples of the diene rubber include isoprene skeleton rubber, styrene-butadiene rubber (SBR), butadiene rubber (BR), and chloroprene rubber (CR). Isoprene skeleton rubber is rubber having an isoprene unit as the main skeleton, such as natural rubber (NR) and synthetic isoprene rubber (IR). Among these, the diene rubber preferably includes at least one selected from the group consisting of isoprene skeleton rubber, styrene-butadiene rubber, and butadiene rubber. When the diene rubber includes at least one selected from the group consisting of isoprene skeleton rubber, styrene-butadiene rubber, and butadiene rubber, diene monomers such as isoprene and butadiene that are easily reused are obtainable.

The content of the diene rubber in the crosslinked rubber is not particularly limited and is preferably, for example, in a range from 10 mass% to 100 mass%. From the viewpoint of further improving the yield of butadiene and isoprene, the content of the diene rubber in the crosslinked rubber is more preferably in a range from 30 mass% to 100 mass%.

-- Carbon Black --

The crosslinked rubber may further contain carbon black. The method of decomposing a crosslinked rubber can decompose diene rubber to a lower molecular weight, for example, to liquid polymers and liquid oligomers, by metathesis decomposition with the catalyst represented by the above general formula (1), (2), or (3) in the first decomposition step. Therefore, even when the crosslinked rubber contains carbon black, the carbon black can be easily separated and recovered after the first decomposition step by, for example, solid-liquid separation. By recovering carbon black prior to pyrolysis in the second decomposition step, reuse as high-grade carbon black is possible. Further, even when the crosslinked rubber contains carbon black, the crosslinked rubber may be efficiently decomposed.

The content of carbon black in the crosslinked rubber is not particularly limited and may be, for example, in a

range from 10 parts by mass to 150 parts by mass, and preferably in a range from 30 parts by mass to 120 parts by mass, relative to 100 parts by mass of the diene rubber.

-- Sulfur --

The crosslinked rubber may further contain sulfur. In crosslinked rubber, sulfur is typically present in a form crosslinking diene rubber (as a crosslink for diene rubber), but some sulfur may be free. The method of decomposing a crosslinked rubber can decompose diene rubber to a lower molecular weight, for example, to liquid polymers and liquid oligomers, by metathesis decomposition with the catalyst represented by the above general formula (1), (2), or (3) in the first decomposition step. Therefore, even when the crosslinked rubber contains sulfur, the sulfur can be easily recovered after the first decomposition step by, for example, solid-liquid separation. Recovery of sulfur prior to pyrolysis in the second decomposition step allows the sulfur to be reused. Further, even when the crosslinked rubber contains sulfur, the crosslinked rubber can be efficiently decomposed.

The content of sulfur in the crosslinked rubber is not particularly limited and may be, for example, in a range from 0.1 parts by mass to 10 parts by mass, and preferably in a range from 1 part by mass to 5 parts by mass, relative to 100 parts by mass of the diene rubber.

-- Other components --

Aside from diene rubber, carbon black, and sulfur mentioned above, the crosslinked rubber may contain various components normally used in the rubber industry, such as a rubber component other than diene rubber, a filler other than carbon black (silica, calcium carbonate, and the like), a silane coupling agent, an antioxidant, a softener, a processing aid, a resin, a surfactant, an organic acid (stearic acid and the like), zinc oxide (zinc flower), a vulcanization accelerator, a crosslinking agent other than sulfur (peroxide and the like), and the like.

(Catalyst)

In the method of decomposing a crosslinked rubber according to the present embodiment, in the first decomposition step, the crosslinked rubber is decomposed using the catalyst represented by the following general formula (1), (2), or (3):

[Chem. 3]

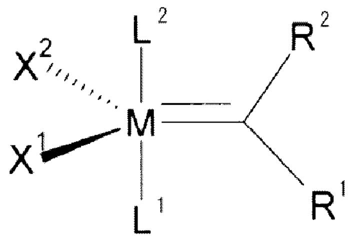

· · · (1)

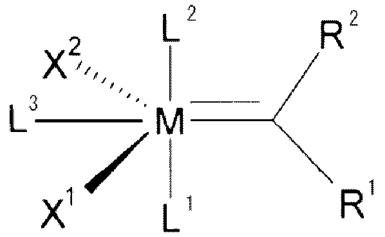

· · · (2)

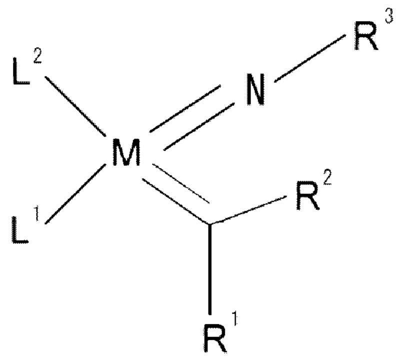

· · · (3)

Catalysts represented by the general formula (1), (2), or (3) can promote metathesis decomposition, and can decompose diene rubber easily (under mild conditions) and quickly.

In the above general formulas (1), (2), and (3), M is ruthenium (Ru), titanium (Ti), molybdenum (Mo), or tungsten (W). Among these, from the viewpoint of promoting the decomposition reaction of diene rubber in the crosslinked rubber, ruthenium is preferred as M.

In the above general formulas (1) and (2), $X^1$ and $X^2$ each independently represent a ligand, preferably an anionic ligand. Examples of $X^1$ and $X^2$ include hydrogen, halogen, pseudohalogen, and, linear or branched, an alkyl group having 1 to 30 carbon atoms, an aryl group having 6 to 24 carbon atoms, an alkoxy group having 1 to 20 carbon atoms, an aryloxy group having 6 to 24 carbon atoms, an alkyl diketonate having 3 to 20 carbon atoms, an aryl diketonate having 6 to 24 carbon atoms, a carboxylate having 1 to 20 carbon atoms, an alkyl sulfonate having 1 to 20 carbon atoms, an aryl sulfonate having 6 to 24 carbon atoms, an alkylthiol group having 1 to 20 carbon atoms, an arylthiol group having 6 to 24 carbon atoms, an alkylsulfonyl group having 1 to 20 carbon atoms, or an alkylsulfinyl group having 1 to 20 carbon atoms.

$X^1$ and $X^2$ may be substituted with one or more further groups, such as halogen (preferably fluorine), an alkyl group having 1 to 10 carbon atoms, an alkoxy group having 1 to 10 carbon atoms, or an aryl group having 6 to 24 carbons, and such groups may also be further substituted with one or more substituents selected from the group consisting of halogen (preferably fluorine), an alkyl group having 1 to 5 carbon atoms, an alkoxy group having 1 to 5 carbons, and a phenyl group.

According to a preferred embodiment, $X^1$ and $X^2$ are the same or different and each is a halogen (in particular,

fluorine, chlorine, bromine, or iodine), a benzoate, a carboxylate having 1 to 5 carbon atoms, an alkyl group having 1 to 5 carbon atoms, a phenoxy group, an alkoxy group having 1 to 5 carbon atoms, an alkylthiol group having 1 to 5 carbon atoms, an arylthiol group having 6 to 24 carbon atoms, an aryl group having 6 to 24 carbon atoms, or an alkylsulfonate having 1 to 5 carbon atoms.

According to a particularly preferred embodiment, $X^1$ and $X^2$ are the same and each is a halogen (in particular, chlorine), $CF_3COO$, $CH_3COO$, $CFH_2COO$, $(CH_3)_3CO$, $(CF_3)_2(CH_3)CO$, $(CF_3)(CH_3)_2CO$, PhO (phenoxy), MeO (methoxy), EtO (ethoxy), tosylate ($p-CH_3-C_6H_4-SO_3$), mesylate (2,4,6-trimethylphenyl), or $CF_3SO_3$ (trifluoromethanesulfonate).

In the above general formulas (1), (2), and (3), $L^1$, $L^2$, and $L^3$ each independently represent a ligand, preferably a neutral (uncharged) electron donor (also referred to as an "electron-donating neutral ligand"). $L^1$, $L^2$, and $L^3$ can be, for example, independently of one another, a phosphine, sulfonated phosphine, phosphate, phosphinic acid ester, phosphonite, arsine, stibine, ether, amine, amide, aryloxy, sulfonate, sulfoxide, carboxyl, nitrosyl, pyridine, thioether, or imidazolidine ligand. $L^1$, $L^2$, and $L^3$ preferably each independently represent an arylphosphine ligand having 6 to 24 carbon atoms, an alkylphosphine ligand having 1 to 10 carbon atoms or a cycloalkylphosphine ligand having 3 to 20 carbon atoms, a sulfonated arylphosphine ligand having 6 to 24 carbon atoms or a sulfonated alkylphosphine ligand having 1 to 10 carbon atoms, an aryl phosphinic acid ester ligand having 6 to 24 carbon atoms or an alkyl phosphinic acid ester ligand having 1 to 10 carbon atoms, an aryl phosphonite ligand having 6 to 24 carbon atoms or an alkyl phosphonite ligand having 1 to 10 carbon atoms, an aryl phosphoric acid ligand having 6 to 24 carbon atoms or an alkyl phosphoric acid ligand having 1 to 10 carbon atoms, an arylarsine ligand having 6 to 24 carbon atoms or an alkylarsine ligand having 1 to 10 carbon atoms, an arylamine ligand having 6 to 24 carbon atoms or an alkylamine ligand having 1 to 10 carbon atoms, a pyridine ligand, an arylsulfoxide ligand having 6 to 24 carbon atoms or an alkylsulfoxide ligand having 1 to 10 carbon atoms, an arylether ligand having 6 to 24 carbon atoms or an alkylether ligand having 1 to 10 carbon atoms, or an arylamide ligand having 6 to 24 carbon atoms or an alkylamide ligand having 1 to 10 carbon atoms, each of which may be substituted with a phenyl group, and the phenyl group may also be optionally further substituted with a halogen, an alkyl group having 1 to 5 carbon atoms, or an alkoxy group having 1 to 5 carbon atoms.

For example, the term "phosphine" includes $PPh_3$, $P(p\text{-}Tol)_3$, $P(o\text{-}Tol)_3$, $PPh(CH_3)_2$, $P(CF_3)_3$, $P(p\text{-}FC_6H_4)_3$, $P(p\text{-}CF_3C_6H_4)_3$, $P(C_6H_4\text{-}SO_3Na)_3$, $P(CH_2C_6H_4\text{-}SO_3Na)_3$, $P(isopropyl)_3$, $P(CHCH_3(CH_2CH_3))_3$, $P(cyclopentyl)_3$, $P(cyclohexyl)_3$, $P(neopentyl)_3$, and $P(neophenyl)_3$.

The term "phosphinic acid ester" includes, for example, triphenyl phosphinic acid, tricyclohexyl phosphinic acid, triisopropyl phosphinic acid, and methyl diphenyl phosphinic acid.

The term "phosphoric acid ester" includes, for example, triphenyl phosphoric acid, tricyclohexyl phosphoric acid, tri-tert-butyl phosphoric acid, triisopropyl phosphoric acid, and methyl diphenyl phosphoric acid.

The term "stibine" includes, for example, triphenylstibine, tricyclohexylstibine, and trimethylstibine.

The term "aryloxy" includes, for example, 2-tert-butyl-4,5-dimethylphenyloxy.

The term "sulfonate" includes, for example, trifluoromethanesulfonate, tosylate, and mesylate.

The term "sulfoxide" includes, for example, $(CH_3)_2S(=O)$ and $(C_6H_5)_2S=O$.

The term "thioether" includes, for example, $CH_3SCH_3$, $C_6H_5SCH_3$, $CH_3OCH_2CH_2SCH_3$, and tetrahydrothiophene.

The term "pyridine" includes, for example, pyridine, picolines ($\alpha$-, $\beta$-, and $\gamma$-picolines), lutidines (2,3-, 2,4-, 2,5-, 2,6-, 3,4- and 3,5-lutidines), collidine (2,4,6-trimethylpyridine), trifluoromethylpyridine, phenylpyridine, 4-(dimethylamino) pyridine, chloropyridines, bromopyridines, nitropyridines, quinoline, pyrimidine, pyrrole, imidazole, and phenylimidazole.

In imidazolidine ligands, the hydrogen bonded to the carbon atom or nitrogen atom of the imidazolidine ring may be substituted with, linear or branched, an alkyl group having 1 to 30 carbon atoms, a cycloalkyl group having 3 to 20 carbon atoms, an alkenyl group having 2 to 20 carbon atoms, an alkynyl group having 2 to 20 carbon atoms, an aryl group having 6 to 24 carbon atoms, a carboxylate having 1 to 20 carbon atoms, an alkoxy group having 1 to 20 carbon atoms, an alkenyloxy group having 2 to 20 carbon atoms, an alkynyloxy group having 2 to 20 carbon atoms, an aryloxy group having 6 to 20 carbon atoms, an alkoxycarbonyl group having 2 to 20 carbon atoms, an alkylthio group having 1 to 20 carbon atoms, an arylthio group having 6 to 20 carbon atoms, an alkylsulfonyl group having 1 to 20 carbon atoms, an alkylsulfonate group having 1 to 20 carbon atoms, an arylsulfonate having 6 to 20 carbon atoms, or an alkylsulfinyl group having 1 to 20 carbon atoms.

In the above general formula (1), (2), and (3), $R^1$, $R^2$, and $R^3$ each independently represent hydrogen, an alkyl group, a cycloalkyl group, an alkenyl group, an alkynyl group, an aryl group, an aralkyl group, a carboxylate group, an alkoxy group, an alkenyloxy group, an alkynyloxy group, an aryloxy group, an alkoxycarbonyl group, an alkylamino group, an alkylthio group, an arylthio group, an alkylsulfonyl group, or an alkylsulfinyl group, and these groups may be substituted by one or more alkyl groups, halogens, alkoxy groups, aryl groups, or heteroaryl groups,

Further, as an alkyl group, an alkyl group having 1 to 30 carbon atoms is preferred; as a cycloalkyl group, a cycloalkyl group having 3 to 20 carbon atoms is preferred; as an alkenyl group, an alkenyl group having 2 to 20 carbon atoms is preferred; as an alkynyl group, an alkynyl group having 2 to 20 carbon atoms is preferred; as an aryl group, an aryl

group having 6 to 24 carbon atoms is preferred; as an aralkyl group, an aralkyl group having 7 to 24 carbon atoms is preferred; as a carboxylate group, a carboxylate group having 1 to 20 carbon atoms is preferred; as an alkoxy group, an alkoxy group having 1 to 20 carbon atoms is preferred; as an alkenyloxy group, an alkenyloxy group having 2 to 20 carbon atoms is preferred; as an alkynyloxy group, an alkynyloxy group having 2 to 20 carbon atoms is preferred; as an aryloxy group, an aryloxy group having 6 to 24 carbon atoms is preferred; as an alkoxycarbonyl group, an alkoxycarbonyl group having 2 to 20 carbon atoms is preferred; as an alkylamino group, an alkylamino group having 1 to 30 carbon atoms is preferred; as an alkylthio group, an alkylthio group having 1 to 30 carbon atoms is preferred; as an arylthio group, an arylthio group having 6 to 24 carbon atoms is preferred; as an alkylsulfonyl group, an alkylsulfonyl group having 1 to 20 carbon atoms is preferred; and as an alkylsulfinyl group, an alkylsulfinyl group having 1 to 20 carbon atoms is preferred.

According to an embodiment, one of $R^1$ and $R^2$ is hydrogen and the other is an alkyl having 1 to 20 carbon atoms, a cycloalkyl having 3 to 10 carbon atoms, an alkenyl having 2 to 20 carbon atoms, an alkynyl having 2 to 20 carbon atoms, an aryl having 6 to 24 carbon atoms, an aralkyl having 7 to 24 carbon atoms, a carboxylate having 1 to 20 carbon atoms, an alkoxy having 1 to 20 carbon atoms, an alkenyloxy having 2 to 20 carbon atoms, an alkynyloxy having 2 to 20 carbon atoms, an aryloxy having 6 to 24 carbon atoms, an alkoxycarbonyl having 2 to 20 carbon atoms, an alkylamino having 1 to 30 carbon atoms, an alkylthio having 1 to 30 carbon atoms, an arylthio having 6 to 24 carbon atoms, an alkylsulfonyl having 1 to 20 carbon atoms, or an alkylsulfinyl having 1 to 20 carbon atoms, each of which may be substituted with one or more alkyl groups, halogens, alkoxy groups, aryl groups, or heteroaryl groups.

In the above general formulas (1), (2), and (3), $L^1$ and $L^2$ may bond with each other to form a ring. A ring formed by $L^1$ and $L^2$ bonded together may be aliphatic or aromatic, and may optionally be substituted to include one or more hetero atoms. Examples of hetero atoms include oxygen, sulfur, nitrogen, phosphorus, and the like.

In the above general formulas (1), (2), and (3), $R^1$ and $R^2$ may bond with each other to form a ring. A ring formed by $R^1$ and $R^2$ bonded together with a common carbon atom to which they are bonded may be aliphatic or aromatic, and $R^1$ and $R^2$ may optionally be substituted to include one or more hetero atoms.

In the above general formulas (1), (2), and (3), $L^1$ and $R^1$ may bond with each other to form a ring. A ring formed by $L^1$ and $R^1$ bonded together may be aliphatic or aromatic, and may optionally be substituted to include one or more hetero atoms. Examples of hetero atoms include oxygen, sulfur, nitrogen, phosphorus, and the like.

As the catalyst represented by the above general formula (1), a catalyst represented by one of the following structural formulas (1-1) to (1-3) is preferred:

[Chem. 4]

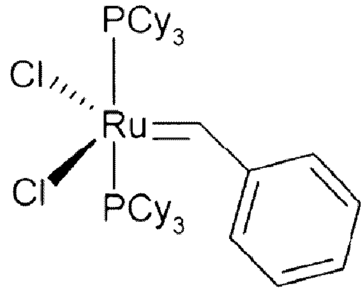

· · · (1-1)

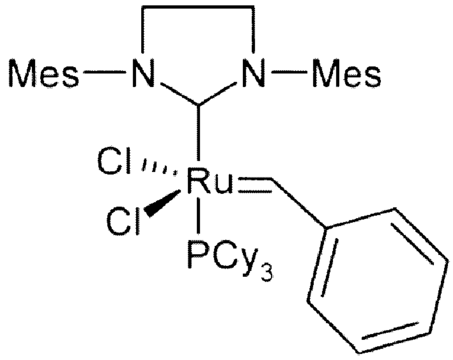

· · · (1-2)

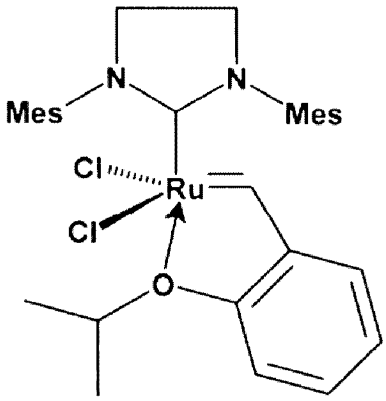

· · · (1-3)

[In the formulas, Cy represents a cyclohexyl group and Mes represents a mesityl group (also called a "2,4,6-trimethylphenyl group").]

A catalyst of structural formula (1-1) is called a first generation Grubbs catalyst, a catalyst of structural formula (1-2) is called a second generation Grubbs catalyst, and a catalyst of structural formula (1-3) is called a second generation Hoveyda-Grubbs catalyst. The decomposition reaction (metathesis decomposition) of diene rubber in the crosslinked rubber proceeds more rapidly when a catalyst represented by one of the structural formulas (1-1) to (1-3) is used.

As the catalyst represented by the above general formula (2), a catalyst represented by the following structural formula (2-1) is preferred:

[Chem. 5]

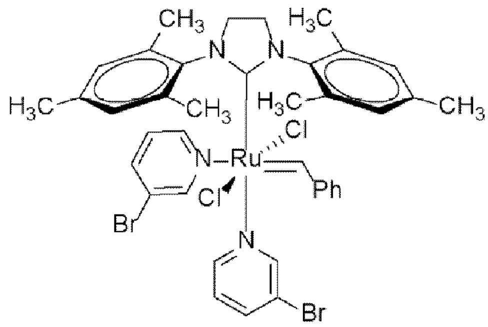

· · · (2-1)

A catalyst of structural formula (2-1) is called a third generation Grubbs catalyst. The decomposition reaction (metathesis decomposition) of diene rubber in the crosslinked rubber proceeds more rapidly when a catalyst represented by the structural formula (2-1) is used.

As the catalyst represented by the above general formula (3), an example is a catalyst represented by the following structural formula (3-1):

[Chem. 6]

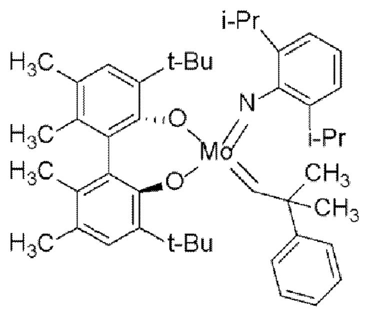

· · · (3-1)

The amount of catalyst used is preferably 0.1 parts by mass or more, more preferably 1 part by mass or more, and preferably 10 parts by mass or less, more preferably 8 parts by mass or less, relative to 100 parts by mass of the diene rubber. When the amount of catalyst used is 0.1 parts by mass or more per 100 parts by mass of the diene rubber, the decomposition reaction of the diene rubber progresses further, and in terms of cost, the amount of catalyst used is preferably 10 parts by mass or less per 100 parts by mass of the diene rubber.

(Reaction conditions and the like)

The first decomposition step is preferably carried out at a temperature of 20°C or more and 200°C or less. Performing the first decomposition step at 20°C or more improves speed of the decomposition reaction of diene rubber in the crosslinked rubber, and performing the first decomposition step at 200°C or less inhibits decomposition of the catalyst represented by the above general formula (1), (2), or (3) (metathesis catalyst), and after the decomposition, the retention rate (selectivity) of the monomer skeleton of diene rubber in the crosslinked rubber is improved. The first decomposition step is more preferably performed at 25°C or more from the viewpoint of improving the decomposition reaction rate of the diene rubber, and more preferably performed at 100°C or less from the viewpoint of inhibiting decomposition of the metathesis catalyst and improving selectivity for a product retaining a monomer skeleton.

The first decomposition step may be performed at any pressure, and may be performed at reduced pressure, at normal pressure, or at increased pressure. As an example, reaction pressure is preferably 1 kPa to 10 MPa, more preferably 10 kPa to 1 MPa, and even more preferably 50 kPa to 500 kPa.

In the method of decomposing a crosslinked rubber according to the present embodiment, the catalyst represented by the general formula (1), (2), or (3) described above may be dissolved in a solvent, and the crosslinked rubber may be immersed in a solvent for the first decomposition step. The catalyst acting on the crosslinked rubber in the solvent facilitates the decomposition reaction of diene rubber in the crosslinked rubber.

Any solvent that does not inhibit the decomposition reaction may be used as the solvent, such as ethers, an aliphatic hydrocarbon, an alicyclic hydrocarbon, an aromatic hydrocarbon (aromatic solvent), and the like. More

specifically, tetrahydrofuran (THF), hexane, cyclohexane, pentane, cyclopentane, toluene, and xylene are preferred solvents, and toluene and tetrahydrofuran are even more preferred. When the solvent is selected from tetrahydrofuran, hexane, cyclohexane, pentane, cyclopentane, toluene, and xylene, the decomposition reaction of diene rubber in the crosslinked rubber is further facilitated.

The amount of solvent used is preferably 10 mL or more, more preferably 50 mL or more, and preferably 500 mL or less, more preferably 200 mL or less, per 1 g of crosslinked rubber. When the amount of solvent used is 10 mL or more per 1 g of crosslinked rubber, the decomposition reaction of diene rubber in the crosslinked rubber progresses further, and in terms of cost, the amount of solvent used is preferably 500 mL or less per 1 g of crosslinked rubber.

The first decomposition step may be performed in the presence of a chain transfer agent (CTA). Here, examples of chain transfer agents include cis-1,4-diacetoxy-2-butene, cis-1,4-dibenzyloxy-2-butene, and the like.

The amount of chain transfer agent used is preferably in a range of 1 mol to 100 mol per 1 mol of the catalyst.

(Decomposition product (intermediate decomposition product))

In the method of decomposing a crosslinked rubber according to the present embodiment, the first decomposition step yields liquid oligomers, liquid polymers, and the like as a decomposition product.

In the method of decomposing a crosslinked rubber according to the present embodiment, the rubber component of the crosslinked rubber contains an isoprene unit and/or a butadiene unit, the total fraction of the isoprene unit and the butadiene unit in the rubber component is 40 mass% or more, and total mass (A) of the isoprene unit and the butadiene unit in the rubber component and mass (B) of aromatic compounds derived from the isoprene unit and/or the butadiene unit in the rubber component of the decomposition product obtained by the first decomposition step preferably satisfy the relationship of the following expression (1):

$$B/A \times 100 \leq 50 \text{ (mass\%)} \cdots (1)$$

[In the expression, A is the total mass of the isoprene unit and the butadiene unit in the rubber component of the crosslinked rubber, and B is the mass of the aromatic compounds derived from the isoprene unit and/or the butadiene unit in the rubber component of the crosslinked rubber, in the decomposition product obtained by the first decomposition step.]

In the method of decomposing a crosslinked rubber according to the present embodiment, the first decomposition step is performed using a catalyst represented by the above general formula (1), (2), or (3), which may prevent aromatization of the decomposition product. The ratio of the mass (B) of aromatic compounds derived from the isoprene unit and/or the butadiene unit in the rubber component of the crosslinked rubber to the total mass (A) of the isoprene unit and the butadiene unit in the rubber component of the crosslinked rubber ($B/A \times 100$) in the decomposition product obtained by the first decomposition step being 50 mass% or less means that aromatization of the decomposition product is suppressed. In this case, the yield of diene monomers that are easily reused such as isoprene and butadiene is improved. From the viewpoint of suppressing aromatization of the decomposition product, the ratio ($B/A \times 100$) is more preferably 40 mass% or less.

When the rubber component of the crosslinked rubber contains an aromatic compound unit (such as a styrene unit), the mass of aromatic compounds derived from the aromatic compound unit in the rubber component of the crosslinked rubber is subtracted from the mass of aromatic compounds in the decomposition product obtained by the first decomposition step in the calculation of the left side of expression (1). Further, the ratio ($B/A \times 100$) can be controlled, for example, by reaction conditions such as reaction temperature and reaction time in the first decomposition step.

In the method of decomposing a crosslinked rubber according to the present embodiment, the liquid component of the decomposition product obtained by the first decomposition step is oligomers having a weight-average molecular weight preferably from 100 to 50,000, more preferably from 400 to 12,000, even more preferably from 400 to 5,000, and even more preferably from 400 to 1,500. By decomposing the crosslinked rubber (rubber component in the crosslinked rubber) to oligomers having a weight-average molecular weight preferably from 100 to 50,000, more preferably from 400 to 12,000, even more preferably from 400 to 5,000, and even more preferably from 400 to 1,500 via the first decomposition step, the yield of monomers via the second decomposition step described below can be further improved.

In the method of decomposing a crosslinked rubber according to the present embodiment, in the first decomposition step, 80 mass% or more of diene rubber in the crosslinked rubber is preferably decomposed to diene oligomers having a weight-average molecular weight of 100 to 50,000, and more preferably decomposed to diene oligomers having a weight-average molecular weight of 300 to 10,000. By decomposing 80 mass% or more of diene rubber in the crosslinked rubber to diene oligomers having a weight-average molecular weight of 100 to 50,000 via the first decomposition step, the yield of diene monomers via the second decomposition step described below can be further improved. From the viewpoint of the yield of diene monomers after the second decomposition step, more preferably 85 mass% or more of diene rubber in the crosslinked rubber is decomposed to diene oligomers having a weight-average molecular weight of 100 to 50,000 via

the first decomposition step.

Hereinafter, oligomers are defined as having a weight-average molecular weight of 50,000 or less and containing two or more monomer units. Further, diene oligomers are oligomers that contain two or more units derived from diene monomers (diene units). Further, weight-average molecular weight (Mw) may be measured by gel permeation chromatography (GPC).

<Second decomposition step>

The method of decomposing a crosslinked rubber according to the present embodiment includes a second decomposition step of pyrolyzing a decomposition product obtained by the first decomposition step under an inert gas atmosphere and in the absence of a catalyst at a temperature of 300°C or more and 450°C or less.

(Reaction conditions and the like)

By performing the second decomposition step at 300°C or more, the rate of decomposition reaction of a decomposition product obtained by the first decomposition step is increased and the yield of monomers is improved, and by performing the second decomposition step at 450°C or less, gasification and aromatization of the decomposition product can be significantly suppressed and selectivity for a product retaining a monomer skeleton is significantly improved. The second decomposition step is preferably performed at 320°C or more from the viewpoint of improving the decomposition reaction rate and monomer yield, and preferably performed at 380°C or less from the viewpoint of suppressing gasification and aromatization of the decomposition product.

The second decomposition step is performed under an inert gas atmosphere. By performing the second decomposition step under an inert gas atmosphere, oxidation and reduction of a decomposition product can be suppressed, in particular hydrogenation of double bonds in monomers in the decomposition product. Examples of inert gases include nitrogen, carbon dioxide, argon, helium, and the like.

To perform the second decomposition step under an inert gas atmosphere, for example, when a batch reactor is used, the atmosphere charged into the reactor may be an inert gas, and when a flow reactor is used, the atmosphere circulated through the reactor may be an inert gas. Although hydrogen may be produced during the second decomposition step, the atmosphere of the second decomposition step does not take into account hydrogen produced.

The second decomposition step may be performed at any pressure, and may be performed at reduced, normal, or increased pressure, and is preferably performed under reduced pressure or normal pressure. As an example, reaction pressure of the second decomposition step is preferably 1000 kPa to 67 kPa. By performing the second decomposition step under reduced pressure or normal pressure, polymerization (repolymerization) of monomers in decomposition product may be suppressed.

The reaction time of the second decomposition step is not particularly limited. As an example, the reaction time for the second decomposition step is preferably from 3 min to 60 min, and more preferably from 5 min to 30 min.

The second decomposition step is performed in the absence of a catalyst (that is, no catalyst is used). The use of no catalyst in the second decomposition step reduces costs. Here, the absence of a catalyst means that there is no catalyst present in the reaction of the second decomposition step that acts to promote the decomposition reaction.

(Decomposition product)

In the method of decomposing a crosslinked rubber according to the present embodiment, the second decomposition step yields monomers (in particular, diene monomers) and the like as a decomposition product.

In the method of decomposing a crosslinked rubber according to the present embodiment, the decomposition product obtained by the second decomposition step preferably contains hydrocarbon compounds having 5 or less carbon atoms and limonene (in particular, hydrocarbon compounds having 2 to 4 carbon atoms, isoprene, and limonene), at a fraction of 15 mass% or more, more preferably 20 mass% or more, and even more preferably 25 mass% or more. Increased yield of hydrocarbon compounds having 5 or less carbon atoms and limonene (in particular, hydrocarbon compounds having 2 to 4 carbon atoms, isoprene, and limonene) increases the yield of reusable monomers, further improving the economic and environmental value of the decomposition method. From the viewpoint of yield of reusable monomers, decomposition product obtained by the second decomposition step more preferably contains 40 mass% or more of hydrocarbon compounds having 5 or less carbon atoms.

Here, the total amount of hydrocarbon compounds having 5 or less carbon atoms and limonene (in particular, hydrocarbon compounds having 2 to 4 carbon atoms, isoprene, and limonene) in the decomposition product obtained by the second decomposition step may depend on the reaction conditions described above as well as the reaction apparatus used. According to the present embodiment, a suitable range of the total amount of hydrocarbon compounds having 5 carbon atoms or less and limonene is a suitable range when the reaction apparatus described in the EXAMPLES section

below is used, and the total amount of hydrocarbon compounds having 5 carbon atoms or less and limonene may be even larger when a more suitable reaction apparatus is used.

Further, as a decomposition product, hydrocarbon compounds having 5 or less carbon atoms vary depending on the type of rubber component in the crosslinked rubber to be decomposed, examples include 1,3-butadiene, isoprene, 1,3-pentadiene, 2,3-dimethyl-1,3-butadiene, and the like, and 1,3-butadiene and isoprene are preferred. The fraction of hydrocarbon compounds having 5 or less carbon atoms in a decomposition product may be controlled, for example, by reaction conditions such as reaction temperature and reaction time in the second decomposition step.

<Other features>

In addition to the first decomposition step and the second decomposition step described above, the method of decomposing a crosslinked rubber according to the present embodiment may include another step. Examples of such a step include a crosslinked rubber pretreatment step (for example, shredding and granulating steps), and the like.

When the crosslinked rubber contains carbon black, a step of recovering carbon black from a decomposition product (intermediate decomposition product) is preferably included between the first decomposition step and the second decomposition step.

Further, the method of decomposing a crosslinked rubber according to the present embodiment may be performed in a batch reactor or in a flow reactor.

Further, decomposition products after a decomposition reaction may be separated and recovered by filtration, distillation, and the like, recovered by precipitation using poor solvents, and the like, and may be reused. Further, diene monomers ultimately obtained from the crosslinked rubber may be reused as raw materials for diene rubber (polymers).

EXAMPLES

This disclosure is described in more detail below with reference to Examples. The present disclosure is not limited in any way by the Examples.

<Molecular weight measurement>

Polystyrene-equivalent weight-average molecular weight (Mw), number-average molecular weight (Mn), and molecular weight distribution (D=Mw/Mn) of decomposition products (intermediate decomposition products) were determined by gel permeation chromatography (hereinafter also referred to as GPC) using polystyrene monodisperse as a standard (pump unit: LC-20AB produced by Shimadzu Corporation, column: a combination of KF-803 and KF-804 produced by Showa Denko K.K. or a combination of G2000HXL and G4000HXL produced by Tosoh Corporation, analysis system: LabSolutions produced by Shimadzu Corporation, eluent: tetrahydrofuran). The measurement temperature was 40°C.

<Decomposition rate of first decomposition step>

Decomposition rate of the first decomposition step was calculated based on the following formula, by recovering post-reaction unreacted solid-state rubber, measuring weight, and using a ratio to weight of pre-reaction rubber. The decomposition rate was defined as 100% when no solid-state rubber remained at all after the reaction.

Decomposition rate of first decomposition step (%) = {1 - (post-reaction unreacted solid-state rubber weight / pre-reaction rubber weight)} $\times$ 100

<Structural formula of catalyst used>

The structural formula of the catalyst used in the first decomposition step was as follows.

[Chem. 7]

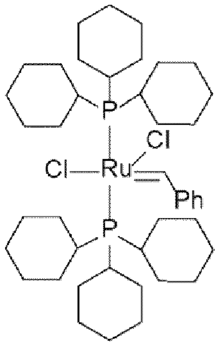

G1

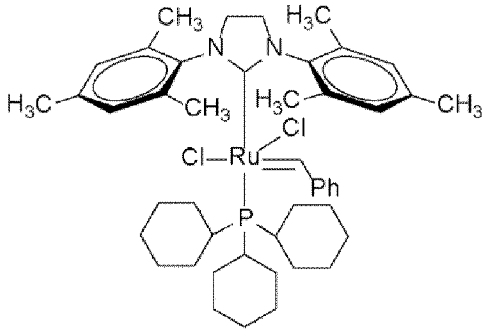

G2

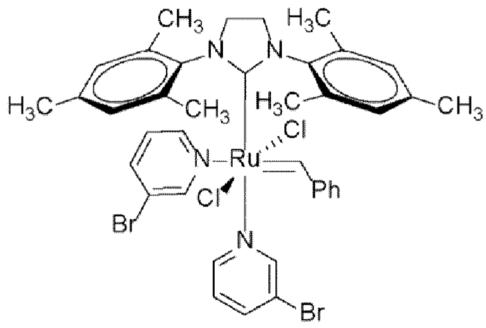

G3

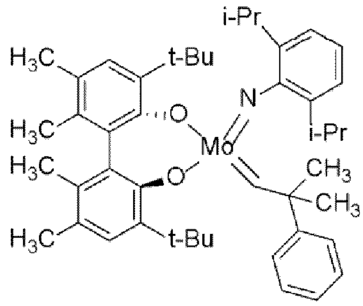

Mo cat

<Preparation of crosslinked rubber samples>

(Crosslinked rubber sample A)

A rubber composition was prepared by blending, relative to 100.1 parts by mass of natural rubber, 50.0 parts by mass of carbon black, 1.0 part by mass of antioxidant 6PPD [N-phenyl-N'-(1,3-dimethylbutyl)-p-phenylenediamine], 2.0 parts by mass of stearic acid, 2.5 parts by mass of zinc oxide, 1.5 parts by mass of vulcanization accelerator (N-cyclohexyl-2-benzothiazolesulfenamide), and 4.5 parts by mass of sulfur, and the rubber composition was heated and crosslinked to prepare crosslinked rubber.

The resulting crosslinked rubber samples were shredded so that each side was approximately 2 mm to 6 mm. Further, crosslinked rubber samples were also prepared by shredding and further freezing and granulation (approximately 1 mm or less per side).

(Crosslinked rubber sample B)

A rubber composition was prepared by blending, relative to 100.0 parts by mass of natural rubber, 50.0 parts by mass of carbon black, 2.0 parts by mass of antioxidant 6PPD [N-phenyl-N'-(1,3-dimethylbutyl)-p-phenylenediamine], 1.0 part by mass of antioxidant trimethyldihydroquinoline polymer, 2.0 parts by mass of stearic acid, 2.25 parts by mass of zinc oxide, 0.6 parts by mass of vulcanization accelerator (N-cyclohexyl-2-benzothiazolesulfenamide), and 1.8 parts by mass of sulfur, and the rubber composition was heated and crosslinked to prepare crosslinked rubber.

The crosslinked rubber was shredded and then further frozen and granulated to prepare crosslinked rubber samples (approximately 1 mm or less per side).

<Example of first decomposition step>

(Example 1)

To 0.125 g of the crosslinked rubber sample A (sample that was shredded only), 10 mL of tetrahydrofuran (THF) was added and stirred at 25°C for 24 h to swell the rubber. 24 mg of second generation Grubbs catalyst (G2) was added and the mixture was stirred at 25°C for another 24 h for metathesis decomposition reaction. After the reaction, reaction was stopped by adding 10 mL of methanol and 2.5 mL of ethyl vinyl ether. No rubber sample remained after the reaction, and the decomposition rate of the rubber sample was calculated to be 100%. The solvent was then removed under reduced pressure to obtain liquid polymer and carbon black. A portion of the mixture was taken, tetrahydrofuran was added, carbon black was removed by filtration using a syringe filter, and molecular weight of the liquid polymer was determined by GPC. Results are listed in Table 1.

(Examples 2 to 10)

Reaction conditions (type and amount of catalyst, type and amount of solvent, and reaction scale) were changed, and the reaction of the first decomposition step was carried out as per Example 1. Results are listed in Table 1.

(Example 11)

To 0.125 g of the crosslinked rubber sample A (sample frozen and granulated after shredding), 10 mL of tetrahydrofuran and 24 mg of second generation Grubbs catalyst (G2) were added and stirred at 25°C for 24 h for metathesis decomposition reaction. After the reaction, reaction was stopped by adding 10 mL of methanol and 2.5 mL of ethyl vinyl ether. No rubber sample remained after the reaction, and the decomposition rate of the rubber sample was calculated to be 100%. The solvent was then removed under reduced pressure to obtain liquid polymer and carbon black. A portion of the mixture was taken, tetrahydrofuran was added, carbon black was removed by filtration using a syringe filter, and molecular weight of the liquid polymer was determined by GPC. Results are listed in Table 1.

(Example 12)

To 0.125 g of the crosslinked rubber sample A (sample that was shredded only), 10 mL of tetrahydrofuran was added and stirred at room temperature for 24 h to swell the rubber. Subsequently, 40 mg of cis-1,4-diacetoxy-2-butene was added as a chain transfer agent (CTA), 24 mg of second generation Grubbs catalyst (G2) was added, and metathesis decomposition was carried out for 24 h. After the reaction, reaction was stopped by adding 10 mL of methanol and 2.5 mL of ethyl vinyl ether. No rubber sample remained after the reaction, and the decomposition rate of the rubber sample was calculated to be 100%. The solvent was then removed under reduced pressure to obtain liquid polymer and carbon black. A portion of the reaction mixture was taken, tetrahydrofuran was added, carbon black was removed by filtration using a syringe filter, and molecular weight of the liquid polymer was determined by GPC. Results are listed in Table 1.

(Example 13)

To 0.125 g of the crosslinked rubber sample B (sample frozen and granulated after shredding), 10 mL of tetrahydrofuran and 8 mg of second generation Grubbs catalyst (G2) were added and stirred at 25°C for 24 h for metathesis decomposition reaction. No rubber sample remained after the reaction, and the decomposition rate of the rubber sample was calculated to be 100%. The reaction solution was diluted with tetrahydrofuran, carbon black was separated by centrifugation, and carbon black was washed with tetrahydrofuran. The first supernatant and the washing solution were combined and concentrated under reduced pressure to obtain liquid polymer. A portion was sampled to determine molecular weight of the liquid polymer by GPC. Results are listed in Table 1.

(Examples 14 to 22)

Reaction conditions (amount of catalyst, type and amount of solvent, and reaction scale) were changed, and the reaction of the first decomposition step was carried out as per Example 13. Results are listed in Table 1.

[Table 1]

| Example | Rubber sample | Rubber weight 1) (g) | Catalyst | Catalyst amount (mg) | Solvent | Solvent amount (mL) | CTA | CTA amount (mg) | Decomposition rate (%) | Mw | Mn | D |
|---|---|---|---|---|---|---|---|---|---|---|---|---|
| Example 1 | A | 0.125 | G2 | 24 | THF | 10 | - | - | 100 | 610 | 390 | 1.6 |
| Example 2 | A | 0.125 | G1 | 24 | THF | 10 | - | - | 1 | - 2) | - 2) | 2) |
| Example 3 | A | 0.125 | G3 | 24 | THF | 10 | - | - | 100 | 1300 | 450 | 2.9 |
| Example 4 | A | 0.125 | Mo cat | 24 | THF | 10 | - | - | 4 | -2) | -2) | -2) |
| Example 5 | A | 0.125 | G2 | 16 | THF | 10 | - | - | 83 | 650 | 380 | 1.7 |
| Example 6 | A | 0.125 | G2 | 48 | THF | 10 | - | - | 100 | 680 | 400 | 1.7 |
| Example 7 | A | 1.25 | G2 | 24 | THF | 100 | - | - | 100 | 700 | 400 | 1.8 |
| Example 8 | A | 2.5 | G2 | 24 | THF | 200 | - | - | 100 | 820 | 420 | 2.0 |
| Example 9 | A | 0.125 | G2 | 24 | toluene | 10 | - | - | 85 | 580 | 390 | 1.5 |
| Example 10 | A | 0.125 | G2 | 24 | dichloromethane | 10 | - | - | 85 | 650 | 400 | 1.6 |
| Example 11 | A | 0.125 | G2 | 24 | THF | 10 | - | - | 100 | 7000 | 700 | 10 |
| Example 12 | A | 0.125 | G2 | 24 | THF | 10 | cis-1,4-diacetoxy-2-butene | 40 | 100 | 850 | 480 | 1.8 |
| Example 13 | B | 0.125 | G2 | 8 | THF | 5 | - | - | 100 | 8700 | 640 | 13 |

(continued)

| Example | Rubber sample | Rubber weight [1)] (g) | Catalyst | Catalyst amount (mg) | Solvent | Solvent amount (mL) | CTA | CTA amount (mg) | Decomposition rate (%) | Mw | Mn | D |
|---|---|---|---|---|---|---|---|---|---|---|---|---|
| Example 14 | B | 0.625 | G2 | 41 | THF | 25 | - | - | 100 | 3400 | 540 | 6.3 |
| Example 15 | B | 1.25 | G2 | 80 | THF | 50 | - | - | 100 | 4300 | 590 | 7.3 |
| Example 16 | B | 0.125 | G2 | 8 | toluene | 5 | - | - | 100 | 670 | 400 | 1.7 |
| Example 17 | B | 0.125 | G2 | 8 | hexane | 5 | - | - | 100 | 780 | 440 | 1.8 |
| Example 18 | B | 0.125 | G2 | 8 | diethyl ether | 5 | - | - | 100 | 950 | 450 | 2.1 |
| Example 19 | B | 0.125 | G2 | 8 | dichloromethane | 5 | - | - | 100 | 850 | 420 | 2.0 |
| Example 20 | B | 0.125 | G2 | 6 | toluene | 5 | - | - | 100 | 610 | 400 | 1.5 |
| Example 21 | B | 0.125 | G2 | 6 | hexane | 5 | - | - | 100 | 660 | 400 | 1.6 |
| Example 22 | B | 0.125 | G2 | 6 | diethyl ether | 5 | - | - | 100 | 870 | 470 | 1.9 |

(Notes for Table 1)

1) In Examples 1 to 10 and 12, samples of crosslinked rubber were shredded only, and in Examples 11 and 13 to 22, samples of crosslinked rubber were shredded and then frozen and granulated. When frozen and granulated samples were used, the step of swelling the rubber was omitted.
2) GPC not measured.

<Second decomposition step>

(Example 23)

The reaction site of a reaction tube was filled with 0.1 g of the oligomer obtained for Example 1, and upstream and downstream sides were filled with quartz wool. A helium introduction line is provided upstream of the reaction tube, and a cooling trap (60% ethylene glycol aqueous solution, cooled at -20°C) filled with chloroform is provided downstream, and gas passing through the trap is collected by a gas bag.

The second decomposition step is carried out by heating the reaction zone of the reaction tube at 400°C for 30 min under the condition of a helium flow rate of 50 mL/min.

After the reaction is completed, the reaction tube is cooled, products precipitated in the reaction tube and trap are collected, and gas products are collected in the gas bag.

Further, the yield of aromatic components and aliphatic components in the product, as well as the yield of the hydrocarbon compounds having 2 to 4 carbon atoms, isoprene, and limonene in the product, are determined by gas chromatography (GC) to be 21 mass% in total. Further, in this total amount, the mass of hydrocarbon compounds having 5 or less carbon atoms is 70%.

## Claims

**1.** A method of decomposing a crosslinked rubber, the method comprising:

a first decomposition step of decomposing a crosslinked rubber containing a diene rubber, using a catalyst represented by the following general formula (1), (2), or (3):

[Chem. 1]

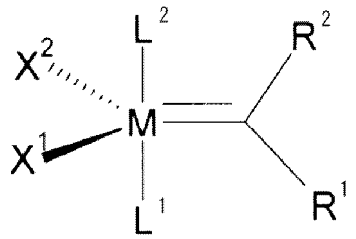

· · · (1)

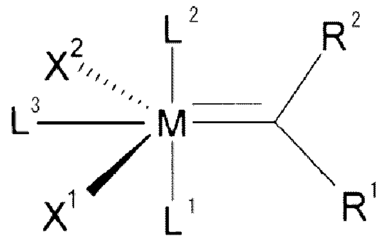

· · · (2)

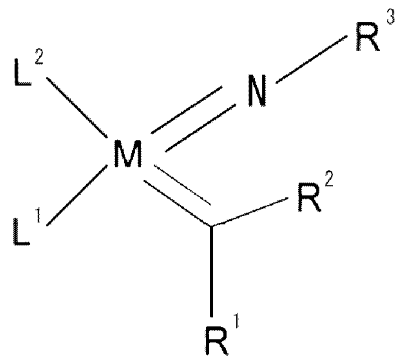

· · · (3)

wherein, in the formulas, M is ruthenium, titanium, molybdenum, or tungsten,
$X^1$ and $X^2$ each independently represent a ligand,
$L^1$, $L^2$, and $L^3$ each independently represent a ligand,
$R^1$, $R^2$, and $R^3$ each independently represent hydrogen, an alkyl group, a cycloalkyl group, an alkenyl group, an alkynyl group, an aryl group, a carboxylate group, an alkoxy group, an alkenyloxy group, an alkynyloxy group, an aryloxy group, an alkoxycarbonyl group, an alkylamino group, an alkylthio group, an arylthio group, an alkylsulfonyl group, or an alkylsulfinyl group, wherein these groups may be substituted by one or more alkyl groups, halogens, alkoxy groups, aryl groups, or heteroaryl groups,
$L^1$ and $L^2$ may bond with each other to form a ring,
$R^1$ and $R^2$ may bond with each other to form a ring, and
$L^1$ and $R^1$ may bond with each other to form a ring; and
a second decomposition step of pyrolyzing a decomposition product obtained by the first decomposition step under an inert gas atmosphere and in the absence of a catalyst at a temperature of 300°C or more and 450°C or less.

**2.** The method of decomposing a crosslinked rubber according to claim 1, wherein a rubber component of the

crosslinked rubber contains an isoprene unit and/or a butadiene unit,

the total fraction of the isoprene unit and the butadiene unit in the rubber component is 40 mass% or more, total mass, A, of the isoprene unit and the butadiene unit in the rubber component and mass, B, of aromatic compounds derived from the isoprene unit and/or the butadiene unit in the rubber component of the decomposition product obtained by the first decomposition step satisfy the relationship of the following expression (1):

$$B/A \times 100 \leq 50 \text{ (mass\%)} \cdots (1)$$

wherein A is the total mass of the isoprene unit and the butadiene unit in the rubber component of the crosslinked rubber, and B is the mass of the aromatic compounds derived from the isoprene unit and/or the butadiene unit in the rubber component of the crosslinked rubber, in the decomposition product obtained by the first decomposition step.

**3.** The method of decomposing a crosslinked rubber according to claim 1 or 2, wherein a liquid component of the decomposition product obtained in the first decomposition step is oligomers having a weight-average molecular weight of 100 to 50,000.

**4.** The method of decomposing a crosslinked rubber according to any one of claims 1 to 3, wherein a decomposition product obtained by the second decomposition step contains 15 mass% or more of a hydrocarbon compound having 5 or less carbon atoms and limonene.

**5.** The method of decomposing a crosslinked rubber according to any one of claims 1 to 4, wherein the first decomposition step is performed at a temperature of 20°C or more and 200°C or less.

**6.** The method of decomposing a crosslinked rubber according to any one of claims 1 to 5, wherein the diene rubber includes at least one rubber selected from the group consisting of isoprene skeleton rubber, styrene-butadiene rubber, and butadiene rubber.

**7.** The method of decomposing a crosslinked rubber according to any one of claims 1 to 6, wherein the crosslinked rubber further contains carbon black.

**8.** The method of decomposing a crosslinked rubber according to any one of claims 1 to 7, wherein the crosslinked rubber further contains sulfur.

## INTERNATIONAL SEARCH REPORT

International application No.
**PCT/JP2023/003873**

**A. CLASSIFICATION OF SUBJECT MATTER**

***C08J 11/18***(2006.01)i; ***B01J 31/22***(2006.01)i; ***B01J 31/24***(2006.01)i; ***C08C 19/08***(2006.01)i; ***C08J 11/12***(2006.01)i
FI: C08J11/18 ZAB; B01J31/22 Z; B01J31/24 Z; C08C19/08; C08J11/12

According to International Patent Classification (IPC) or to both national classification and IPC

**B. FIELDS SEARCHED**

Minimum documentation searched (classification system followed by classification symbols)

C08J11/18; B01J31/22; B01J31/24; C08C19/08; C08J11/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JST7580/JSTChina (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | CN 110922510 A (HUAIAN DADI FLOWER AND WOOD CO., LTD.) 27 March 2020 (2020-03-27)<br>in particular, examples 2, 5 | 1-8 |
| A | Green Chemistry, 2016, vol. 18, pp. 3448-3455<br>entire text, all drawings | 1-8 |
| A | US 5446102 A (BRIDGESTON, CORP.) 29 August 1995 (1995-08-29)<br>in particular, examples 5-12 | 1-8 |
| A | US 2006/0116431 A1 (MCFARLANE, Richard Anthony) 01 June 2006 (2006-06-01)<br>claims, examples | 1-8 |
| A | WO 2014/148612 A1 (NIPPON ZEON CO., LTD.) 25 September 2014 (2014-09-25)<br>claims, examples | 1-8 |
| A | JP 2009-106929 A (LANXESS DEUTSCHLAND GMBH) 21 May 2009 (2009-05-21)<br>claims, examples | 1-8 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 March 2023** | **04 April 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
PCT/JP2023/003873

C. DOCUMENTS CONSIDERED TO BE RELEVANT

| Category* | Citation of document, with indication, where appropriate, of the relevant passages | Relevant to claim No. |
|---|---|---|
| A | Macromolecules, 2001, Vol. 34, pp. 7929-7931<br>entire text, all drawings | 1-8 |
| A | Polymer Degradation and Stability, 2013, vol. 98, pp. 736-742<br>entire text, all drawings | 1-8 |
| A | JP 7-316227 A (ASAHI CHEMICAL INDUSTRIES CO., LTD.) 05 December 1995 (1995-12-05)<br>claims, examples | 1-8 |

Form PCT/ISA/210 (second sheet) (January 2015)

**INTERNATIONAL SEARCH REPORT**
**Information on patent family members**

International application No.
**PCT/JP2023/003873**

| Patent document cited in search report | Publication date (day/month/year) | Patent family member(s) | Publication date (day/month/year) |
|---|---|---|---|
| CN 110922510 A | 27 March 2020 | (Family: none) | |
| US 5446102 A | 29 August 1995 | (Family: none) | |
| US 2006/0116431 A1 | 01 June 2006 | WO 2006/056072 A1<br>CN 101218287 A | |
| WO 2014/148612 A1 | 25 September 2014 | US 2016/0122457 A1<br>claims, examples<br>EP 2977388 A1<br>CN 105102486 A<br>KR 10-2015-0132180 A | |
| JP 2009-106929 A | 21 May 2009 | US 2009/0069516 A1<br>claims, examples<br>EP 2027919 A2<br>CN 101371992 A | |
| JP 7-316227 A | 05 December 1995 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009247241 A